# EUROPEAN PATENT APPLICATION

(11) **EP 4 835 472 A1**
(43) Date of publication of application: **04.11.2026**
(21) Application number: 23962858.9
(22) Date of filing: 29.12.2023
(51) Int. Cl.: G01N 33/53, G01N 33/50

(54) **POINT-OF-CARE TESTING DEVICE**

(71) Applicant: Shenzhen Mindray Animal Medical Technology Co., Ltd., Shenzhen, Guangdong 518110 (CN)
(72) Inventor: WANG, Shengxi, Shenzhen, Guangdong 518110 (CN); LI, Liya, Shenzhen, Guangdong 518110 (CN); DU, Xiansuan, Shenzhen, Guangdong 518110 (CN)
(74) Representative: KIPA AB
(86) International application number: PCT/CN2023/143480
(87) International publication number: WO 2025/138184

(57) **Abstract**

A point-of-care testing device. A control module or a second point-of-care testing module of the point-of-care testing device can be used in combination with at least one first point-of-care testing module; a user can select the first point-of-care testing module having a required point-of-care testing item, so that the first point-of-care testing module and the control module or the second point-of-care testing module form the point-of-care testing device; the control module or the second point-of-care testing module is used for receiving a test request instruction inputted by the user, and on the basis of the test request instruction, sending a control instruction to the corresponding first point-of-care testing module. The control module or the second point-of-care testing module can be used as a main control system to control the one or more first point-of-care testing modules, and these first point-of-care testing modules can simplify the structure and are only used for receiving the control instruction of the control module or the second point-of-care testing module, and achieving a corresponding point-of-care testing operation, thereby being conducive to reducing the size of the whole point-of-care testing device.

## Description

### TECHNICAL FIELD

The disclosure relates to the field of medical devices, and in particular to a structure of a point-of-care testing device for sample testing.

### BACKGROUND

A POCT device, also referred to as a point-of-care testing device, is a device for clinical testing and bedside testing performed beside a patient. The device features speed, ease of use, low overall costs, etc. Common point-of-care testing devices may include, but are not limited to, an immunochromatographic analyzer, a microfluidic biochemical analyzer, a urine dry chemistry analyzer, a blood-gas and electrolyte analyzer, etc.

In a current medical environment, due to a limited area of the test site, the point-of-care testing devices are required to be compact and occupy a small footprint. However, a single current point-of-care testing device usually only has a test function for a corresponding item. For example, an immunochromatographic analyzer can only perform testing for an immunoassay item, and a microfluidic biochemical analyzer can only perform testing for a biochemical item. This would require medical institutions to configure a variety of point-of-care testing devices at the same time. These point-of-care testing devices operate separately, not only greatly occupying the space of the test site, but also increasing the costs of purchasing instruments.

### SUMMARY

### TECHNICAL PROBLEM

A first aspect of the disclosure provides a point-of-care testing device. A control module in the point-of-care testing device may be used in combination with at least one first point-of-care testing module, and the first point-of-care testing module is controlled by the control module.

A second aspect of the disclosure provides a point-of-care testing device. In addition to its own ability to perform a point-of-care testing operation on a sample, a second point-of-care testing module in the point-of-care testing device may be used in combination with at least one first point-of-care testing module, and the first point-of-care testing module is controlled by the second point-of-care testing module.

A third aspect of the disclosure provides a control module of a point-of-care testing device, and the control module may be used to control at least one first point-of-care testing module.

A fourth aspect of the disclosure provides a second point-of-care testing module of a point-of-care testing device. In addition to its own ability to perform a point-of-care testing operation on a sample, the second point-of-care testing module may be used to control at least one first point-of-care testing module.

A fifth aspect of the disclosure provides a first point-of-care testing module of a point-of-care testing device. The first point-of-care testing module may be used in combination with a corresponding control module or second point-of-care testing module and is controlled by the control module or the second point-of-care testing module to complete a testing operation.

### TECHNICAL SOLUTION

According to a first aspect of the disclosure, a point-of-care testing device is provided in an embodiment. The point-of-care testing device includes:
at least one first point-of-care testing module, each of which has a first housing and a first detection assembly, the first detection assembly being mounted on the first housing, and the first detection assembly being configured to perform detection on a sample;
and a control module having a second housing independent of the first housing and a main control unit, the main control unit being mounted on the second housing, where
the main control unit is communicatively connected to each of the first point-of-care testing modules, and is configured to receive a test request instruction input by a user and issue a control instruction to the corresponding first point-of-care testing module according to the test request instruction; and each of the first point-of-care testing modules is capable of completing at least one point-of-care testing operation under the control of the main control unit.

In an embodiment, the first detection assembly includes a sensor configured to perform detection on the sample and obtain a detection signal, the first detection assembly is capable of transmitting the detection signal to the main control unit, and the main control unit processes the detection signal and obtains a detection result; or
the first detection assembly includes a sensor configured to perform detection on the sample and obtain a detection signal, the first point-of-care testing module includes a sub-control unit capable of processing the detection signal, the sub-control unit obtains a detection result based on the detection signal, the sub-control unit is communicatively connected to the main control unit, and the sub-control unit transmits the detection result to the main control unit.

In an embodiment, the first point-of-care testing module has a communication unit and a communication connection line, where the communication connection line has a first communication port and a second communication port, the first communication port is configured to be supplied with a communication signal directly or indirectly output by the control module and output a communication signal to the control module, and the second communication port is electrically connected to the communication unit, such that the communication unit is capable of communicating with the control module.

In an embodiment, at least part of the communication connection line also has a third communication port, the third communication port is electrically connected to the first communication port, and the third communication port is configured to output the communication signal from the first communication port to another first point-of-care testing module and output a communication signal from the another first point-of-care testing module to the first communication port.

In an embodiment, at least part of the first point-of-care testing modules has a power supply unit and a power supply connection line, where the power supply connection line has a first power supply port and a second power supply port, the first power supply port is configured to be connected to a power signal directly or indirectly output by the control module, and the second power supply port is in power supply connection to the power supply unit, to transmit the power signal directly or indirectly output by the control module to the power supply unit.

In an embodiment, at least part of the power supply connection line also has a third power supply port, the third power supply port is in power supply connection to the first power supply port, and the third power supply port is configured to output a power signal of the first power supply port to a next first point-of-care testing module.

In an embodiment, the first communication port of each of the first point-of-care testing modules is communicatively connected in parallel to the control module.

In an embodiment, each of the first point-of-care testing modules has a power supply unit and a power supply connection line, where the power supply connection line has a first power supply port and a second power supply port, the second power supply port is in power supply connection to the power supply unit, and the first power supply port of each of the first point-of-care testing modules is electrically connected in parallel to the control module.

In an embodiment, for the first point-of-care testing module, the first communication port and/or the first power supply port is disposed on a top wall of the first housing.

In an embodiment, for the first point-of-care testing module, the first communication port and the third communication port are disposed opposite each other, with the first communication port located on the top wall of the first housing and the third communication port located on a bottom wall of the first housing; and/or
the first power supply port and the third power supply port are disposed opposite each other, with the first power supply port located on the top wall of the first housing and the third power supply port located on the bottom wall of the first housing.

In an embodiment, the first point-of-care testing module has a first pogo pin interface, and the first pogo pin interface includes the first communication port and the first power supply port.

In an embodiment, the first point-of-care testing module has a second pogo pin interface, and the second pogo pin interface includes the third communication port and the third power supply port.

In an embodiment, the first point-of-care testing module has a communication unit, and each communication unit is connectable to the main control unit via wireless communication.

In an embodiment, the control module is capable of independently communicating with each of the first point-of-care testing modules.

In an embodiment, the control module has an external mounting structure, and the external mounting structure is detachably connected to the corresponding first point-of-care testing module, such that the control module is assemblable with the corresponding first point-of-care testing module to form an integrated structure.

In an embodiment, there are at least two first point-of-care testing modules, at least one of the first point-of-care testing modules has a first mounting structure and a second mounting structure, and the first mounting structure is detachably connectable to the external mounting structure of the control module or the second mounting structure of another first point-of-care testing module.

In an embodiment, a bottom wall of the second housing is provided with the external mounting structure, and the first mounting structure is disposed on the top wall of the first housing of the first point-of-care testing module, such that the control module is capable of being located above the first point-of-care testing module.

In an embodiment, the second mounting structure is disposed on the bottom wall of the first point-of-care testing module for docking with the first mounting structure of another first point-of-care testing module, such that each of the first point-of-care testing modules is stackable vertically with the control module.

In an embodiment, the detachable connection is at least one of snap fit, screw connection, magnetic fixation, fastener fixation, or adhesive bonding.

In an embodiment, the control module and each of the first point-of-care testing modules are stacked into an integrated structure in a non-mechanical connection manner.

In an embodiment, in the integrated structure, the control module is located at the top of the integrated structure.

In an embodiment, the control module and each of the first point-of-care testing modules are stacked vertically;
or the control module and each of the first point-of-care testing modules are stacked in a pyramidal shape;
or the control module and each of the first point-of-care testing modules are stacked in a cubic shape;
or the control module and each of the first point-of-care testing modules are arranged in a straight line.

In an embodiment, the control module has at least one of an external power input interface, a human-machine interaction unit, or a printer.

In an embodiment, at least one of the first point-of-care testing modules has a built-in power supply battery.

In an embodiment, a type of the first point-of-care testing module is at least one of an immunochromatographic detector, a microfluidic biochemical detector, a urine dry chemistry detector, a blood-gas and electrolyte detector, or a blood cell detector.

According to a second aspect of the disclosure, a point-of-care testing device is provided in an embodiment. The point-of-care testing device includes:
at least one first point-of-care testing module, each of which has a first housing and a first detection assembly, the first detection assembly being mounted on the first housing, and the first detection assembly being configured to perform detection on a sample;
and a second point-of-care testing module having a second housing independent of the first housing, a second detection assembly, and a main control unit, the second detection assembly and the main control unit being mounted on the second housing, and the main control unit being communicatively connected to the second detection assembly and controlling, according to a test request instruction input by a user, the second detection assembly to perform detection on the sample, where
the main control unit is further communicatively connected to each of the first point-of-care testing modules, and is configured to receive the test request instruction input by the user and issue a control instruction to the corresponding first point-of-care testing module according to the test request instruction; and each of the first point-of-care testing modules is capable of completing at least one point-of-care testing operation under the control of the main control unit.

In an embodiment, the first detection assembly includes a sensor configured to perform detection on the sample and obtain a detection signal, the first detection assembly is capable of transmitting the detection signal to the main control unit, and the main control unit processes the detection signal and obtains a detection result; or
the first detection assembly includes a sensor configured to perform detection on the sample and obtain a detection signal and a sub-control unit capable of processing the detection signal, the sub-control unit obtains a detection result based on the detection signal, the sub-control unit is communicatively connected to the main control unit, and the sub-control unit transmits the detection result to the main control unit.

In an embodiment, the first point-of-care testing module has a communication unit and a communication connection line, where the communication connection line has a first communication port and a second communication port, the first communication port is configured to be supplied with a communication signal directly or indirectly output by the second point-of-care testing module and output a communication signal to the second point-of-care testing module, and the second communication port is electrically connected to the communication unit, such that the communication unit is capable of communicating with the control module.

In an embodiment, at least part of the communication connection line also has a third communication port, the third communication port is electrically connected to the first communication port, and the third communication port is configured to output the communication signal from the first communication port to another first point-of-care testing module and output a communication signal from the another first point-of-care testing module to the first communication port.

In an embodiment, at least part of the first point-of-care testing modules has a power supply unit and a power supply connection line, where the power supply connection line has a first power supply port and a second power supply port, the first power supply port is configured to be connected to a power signal directly or indirectly output by the second point-of-care testing module, and the second power supply port is in power supply connection to the power supply unit, to transmit the power signal directly or indirectly output by the second point-of-care testing module to the power supply unit.

In an embodiment, at least part of the power supply connection line also has a third power supply port, the third power supply port is in power supply connection to the first power supply port, and the third power supply port is configured to output a power signal of the first power supply port to a next first point-of-care testing module.

In an embodiment, the first communication port of each of the first point-of-care testing modules is communicatively connected in parallel to the second point-of-care testing module.

In an embodiment, each of the first point-of-care testing modules has a power supply unit and a power supply connection line, where the power supply connection line has a first power supply port and a second power supply port, the second power supply port is in power supply connection to the power supply unit, and the first power supply port of each of the first point-of-care testing modules is electrically connected in parallel to the second point-of-care testing module.

In an embodiment, for the first point-of-care testing module, the first communication port and/or the first power supply port is disposed on a top wall of the first housing.

In an embodiment, for the first point-of-care testing module, the first communication port and the third communication port are disposed opposite each other, with the first communication port located on the top wall of the first housing and the third communication port located on a bottom wall of the first housing; and/or
the first power supply port and the third power supply port are disposed opposite each other, with the first power supply port located on the top wall of the first housing and the third power supply port located on the bottom wall of the first housing.

In an embodiment, the first point-of-care testing module has a first pogo pin interface, and the first pogo pin interface includes the first communication port and the first power supply port.

In an embodiment, the first point-of-care testing module has a second pogo pin interface, and the second pogo pin interface includes the third communication port and the third power supply port.

In an embodiment, the first point-of-care testing module has a communication unit, and each communication unit is connectable to the main control unit via wireless communication.

In an embodiment, the second point-of-care testing module is capable of independently communicating with each of the first point-of-care testing modules.

In an embodiment, the second point-of-care testing module has an external mounting structure, and the external mounting structure is detachably connected to the corresponding first point-of-care testing module, such that the second point-of-care testing module is assemblable with the corresponding first point-of-care testing module to form an integrated structure.

In an embodiment, there are at least two first point-of-care testing modules, at least one of the first point-of-care testing modules has a first mounting structure and a second mounting structure, and the first mounting structure is detachably connectable to the external mounting structure of the second point-of-care testing module or the second mounting structure of another first point-of-care testing module.

In an embodiment, a bottom wall of the second housing is provided with the external mounting structure, and the first mounting structure is disposed on the top wall of the first housing of the first point-of-care testing module, such that the second point-of-care testing module is capable of being located above the first point-of-care testing module.

In an embodiment, the second mounting structure is disposed on the bottom wall of the first point-of-care testing module for docking with the first mounting structure of another first point-of-care testing module, such that each of the first point-of-care testing modules is stackable vertically with the second point-of-care testing module.

In an embodiment, the detachable connection is at least one of snap fit, screw connection, magnetic fixation, fastener fixation, or adhesive bonding.

In an embodiment, the second point-of-care testing module and each of the first point-of-care testing modules are stacked into an integrated structure in a non-mechanical connection manner.

In an embodiment, in the integrated structure, the second point-of-care testing module is located at the top of the integrated structure.

In an embodiment, the second point-of-care testing module and each of the first point-of-care testing modules are stacked vertically;
or the second point-of-care testing module and each of the first point-of-care testing modules are stacked in a pyramidal shape;
or the second point-of-care testing module and each of the first point-of-care testing modules are stacked in a cubic shape;
or the second point-of-care testing module and each of the first point-of-care testing modules are arranged in a straight line.

In an embodiment, the second point-of-care testing module has at least one of an external power input interface, a human-machine interaction unit, or a printer.

In an embodiment, at least one of the first point-of-care testing modules has a built-in power supply battery.

In an embodiment, a type of the first point-of-care testing module is at least one of an immunochromatographic detector, a microfluidic biochemical detector, a urine dry chemistry detector, a blood-gas and electrolyte detector, or a blood cell detector.

According to a third aspect of the disclosure, a control module of a point-of-care testing device is provided in an embodiment. The control module has a main control unit, a main communication unit, and a second housing disposed independent of a first point-of-care testing module, where the main control unit and the main communication unit are mounted on the second housing; and the main communication unit is configured to be communicatively connected to a corresponding first point-of-care testing module, and the main control unit is configured to receive a test request instruction input by a user and is capable of issuing a control instruction to the corresponding first point-of-care testing module according to the test request instruction.

In an embodiment, the second housing has an external mounting structure, and the external mounting structure is detachably connectable to the corresponding point-of-care testing module, such that the control module is assemblable with the corresponding point-of-care testing module to form an integrated structure.

According to a fourth aspect of the disclosure, a second point-of-care testing module of a point-of-care testing device is provided in an embodiment. The second point-of-care testing module has a second housing, a second detection assembly, a main communication unit, and a main control unit, where the second detection assembly, the main communication unit, and the main control unit are mounted on the second housing, and the second detection assembly is configured to perform detection on a sample; and the main communication unit is configured to be communicatively connected to a corresponding first point-of-care testing module, and the main control unit is configured to receive a test request instruction input by a user and is capable of issuing a control instruction to the corresponding first point-of-care testing module according to the test request instruction.

In an embodiment, the second housing has an external mounting structure, and the external mounting structure is detachably connectable to the corresponding point-of-care testing module, such that the control module is assemblable with the corresponding point-of-care testing module to form an integrated structure.

According to a fifth aspect of the disclosure, a first point-of-care testing module of a point-of-care testing device is provided in an embodiment. The first point-of-care testing module has a first housing, a communication unit, and a first detection assembly, where the first detection assembly is configured to perform detection on a sample, and the communication unit is configured to be communicatively connected to a main control unit; and
the first detection assembly and the communication unit are mounted on the first housing; and the first housing has a first mounting structure, and the first mounting structure is detachably connectable to the control module or the second point-of-care testing module, such that the first point-of-care testing module is assembled with the control module or the second point-of-care testing module to form an integrated structure.

In an embodiment, the first housing has a second mounting structure capable of extension by connecting another first point-of-care testing module, and the second mounting structure is configured to assemble the first point-of-care testing module and the another first point-of-care testing module into an integrated structure.

In an embodiment, the first mounting structure and the second mounting structure are respectively disposed on two opposite side surfaces of the first housing.

In an embodiment, the first point-of-care testing module has a communication connection line, where the communication connection line has a first communication port and a second communication port, the first communication port is configured to be supplied with a communication signal directly or indirectly output by the second point-of-care testing module and output a communication signal to the second point-of-care testing module, and the second communication port is electrically connected to the communication unit, such that the communication unit is capable of communicating with the main control unit.

In an embodiment, the communication connection line also has a third communication port, the third communication port is electrically connected to the first communication port, and the third communication port is configured to output the communication signal from the first communication port to another first point-of-care testing module and output a communication signal from the another first point-of-care testing module to the first communication port.

In an embodiment, the first point-of-care testing module has a power supply unit and a power supply connection line, where the power supply connection line has a first power supply port and a second power supply port, the first power supply port is configured to be connected to a power signal directly or indirectly output by the control module, and the second power supply port is in power supply connection to the power supply unit, to transmit the power signal directly or indirectly output by the control module to the power supply unit.

In an embodiment, at least part of the power supply connection line also has a third power supply port, the third power supply port is in power supply connection to the first power supply port, and the third power supply port is configured to output a power signal of the first power supply port to a next first point-of-care testing module.

In an embodiment, for the first point-of-care testing module, the first communication port and/or the first power supply port is disposed on a top wall of the first housing.

In an embodiment, for the first point-of-care testing module, the first communication port and the third communication port are disposed opposite each other, with the first communication port located on the top wall of the first housing and the third communication port located on a bottom wall of the first housing; and/or
the first power supply port and the third power supply port are disposed opposite each other, with the first power supply port located on the top wall of the first housing and the third power supply port located on the bottom wall of the first housing.

In an embodiment, the first point-of-care testing module has a first pogo pin interface, and the first pogo pin interface includes the first communication port and the first power supply port.

In an embodiment, the first point-of-care testing module has a second pogo pin interface, and the second pogo pin interface includes the third communication port and the third power supply port.

### BENEFICIAL EFFECTS

According to the point-of-care testing device in the above-mentioned embodiments, the control module of the point-of-care testing device or the second point-of-care testing module may be used in combination with at least one first point-of-care testing module, allowing the user to select a first point-of-care testing module having a required point-of-care testing item, such that the point-of-care testing module and the control module or the second point-of-care testing module form a point-of-care testing device. The control module or the second point-of-care testing module is configured to receive a test request instruction input by the user and issue a control instruction to the corresponding first point-of-care testing module according to the test request instruction. Each first point-of-care testing module is capable of completing at least one point-of-care testing operation under the control of the control module or the second point-of-care testing module. The control module or the second point-of-care testing module may be used as a main control system to control one or more first point-of-care testing modules. The first point-of-care testing modules may be simplified in structure provided that the first point-of-care testing modules may be used only to receive control instructions of the control module or the second point-of-care testing module and implement a corresponding point-of-care testing operation, which helps reduce the size of the entire point-of-care testing device. In addition, only a corresponding first point-of-care testing module may be selected based on a required point-of-care testing function and then combined with the control module or the second point-of-care testing module, helping to increase the diversity and availability of the point-of-care testing device and reduce the waste of the first point-of-care testing module.

The control module of the point-of-care testing device in the above-mentioned embodiments may be used to control at least one first point-of-care testing module, such that the corresponding first point-of-care testing module may be simplified in structure provided that the first point-of-care testing modules may be used only to receive control instructions of the control module and implement a corresponding point-of-care testing operation, which helps reduce the size of the entire point-of-care testing device. In addition, the control module may be combined with different types of first point-of-care testing modules, and a corresponding first point-of-care testing module may be selected based on a required point-of-care testing function, helping to increase the diversity and availability of the point-of-care testing device and reduce the waste of the first point-of-care testing module.

The second point-of-care testing module of the point-of-care testing device in the above-mentioned embodiments may be used to control at least one first point-of-care testing module, such that the corresponding first point-of-care testing module may be simplified in structure provided that the first point-of-care testing modules may be used only to receive control instructions of the second point-of-care testing module and implement a corresponding point-of-care testing operation, which helps reduce the size of the entire point-of-care testing device. In addition, the second point-of-care testing module may be combined with different types of first point-of-care testing modules, and a corresponding first point-of-care testing module may be selected based on a required point-of-care testing function, helping to increase the diversity and availability of the point-of-care testing device and reduce the waste of the first point-of-care testing module.

The first point-of-care testing module of the point-of-care testing device in the above-mentioned embodiments may be used in combination with a corresponding control module or second point-of-care testing module and is controlled by the control module or the second point-of-care testing module to complete a testing operation. The first point-of-care testing module may be used only to receive control instructions of the second point-of-care testing module and implement a corresponding point-of-care testing operation, simplifying its own structure and helping reduce the size of the entire point-of-care testing device.

### BRIEF DESCRIPTION OF THE DRAWINGS

FIG. 1 is a schematic diagram of a vertically stacked and assembled structure of a point-of-care testing device according to an embodiment of the disclosure;
FIG. 2 is a schematic diagram of a horizontally assembled structure of a point-of-care testing device according to an embodiment of the disclosure;
FIG. 3 is a schematic diagram of a triangular assembly structure of a point-of-care testing device according to an embodiment of the disclosure;
FIG. 4 is a schematic diagram of a pyramidal assembly structure of a point-of-care testing device according to an embodiment of the disclosure;
FIG. 5 is a schematic diagram of a cubic assembly structure of a point-of-care testing device according to an embodiment of the disclosure;
FIG. 6 is a schematic structural diagram of a case where an external interface on a control module or a second point-of-care testing module is located on a bottom wall of a second housing according to an embodiment of the disclosure, where in FIG. 1 to FIG. 6, 1 (2) indicates that the component may be either a control module 1 or a second point-of-care testing module 2;
FIG. 7 is a schematic structural diagram of a case where a first communication port and a first power supply port on a first point-of-care testing module are disposed on a top wall of a first housing, and a third communication port and a third power supply port are disposed on a bottom wall of the first housing according to an embodiment of the disclosure;
FIG. 8 is a schematic structural diagram of a case where a first communication port and a first power supply port on a first point-of-care testing module are disposed on a side wall of a first housing, and a third communication port and a third power supply port are disposed on another side wall of the first housing according to an embodiment of the disclosure;
FIG. 9 is a schematic diagram of a communication connection between a control module and one first point-of-care testing module according to an embodiment of the disclosure, where a dashed line only indicates the communication connection between the control module and the first point-of-care testing module, which may be a wired connection, a wireless connection, or both;
FIG. 10 is a schematic diagram of a communication connection between a control module and one first point-of-care testing module according to an embodiment of the disclosure, where a dashed line only indicates the communication connection between the control module and the first point-of-care testing module, which may be a wired connection, a wireless connection, or both;
FIG. 11 is a schematic diagram of a communication connection and a power supply connection between a control module and two first point-of-care testing modules according to an embodiment of the disclosure;
FIG. 12 is a schematic diagram of a communication connection between a second point-of-care testing module and one first point-of-care testing module according to an embodiment of the disclosure, where a dashed line only indicates the communication connection between the second point-of-care testing module and the first point-of-care testing module, which may be a wired connection, a wireless connection, or both;
FIG. 13 is a schematic diagram of a communication connection between a second point-of-care testing module and one first point-of-care testing module according to an embodiment of the disclosure, where a dashed line only indicates the communication connection between the second point-of-care testing module and the first point-of-care testing module, which may be a wired connection, a wireless connection, or both; and
FIG. 14 is a schematic diagram of a communication connection and a power supply connection between a second point-of-care testing module and two first point-of-care testing modules according to an embodiment of the disclosure.

### DETAILED DESCRIPTION OF THE INVENTION

The disclosure will be further described in detail below through specific implementations in conjunction with the accompanying drawings. Associated similar element reference numerals are used for similar elements in different implementations. In the following implementations, many details are described such that the disclosure may be better understood. However, it may be effortlessly appreciated by persons skilled in the art that some of the features may be omitted, or may be substituted by other elements, materials, and methods in different cases. In certain cases, some operations involved in the disclosure are not displayed or described in the specification, which is to prevent a core part of the disclosure from being obscured by too much description. Moreover, for persons skilled in the art, a detailed description of the involved operations is not necessary, and the involved operations can be thoroughly understood according to the description in the specification and general technical knowledge of the art.

In addition, the characteristics, operations, or features described in the specification may be combined in any appropriate manner to form various implementations. In addition, the steps or actions in the method description may also be exchanged or adjusted in order in a way that is obvious to persons skilled in the art. Therefore, the various orders in the specification and the accompanying drawings are merely for the purpose of a clear description of a certain embodiment and are not meant to be a necessary order unless it is otherwise stated that a certain order must be followed.

The serial numbers themselves for the components herein, for example, "first", "second", etc. are merely used to distinguish the described objects, and do not have any sequential or technical meaning. Moreover, as used in the disclosure, "connection" or "coupling", unless otherwise stated, includes both direct and indirect connections (couplings).

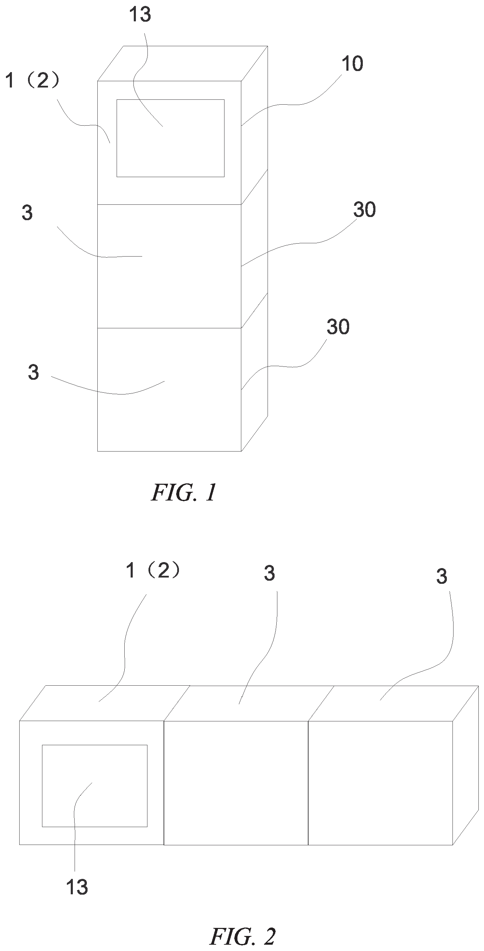

The disclosure provides a point-of-care testing device that may be used to implement at least one point-of-care testing item. Referring to FIG. 1 to FIG. 5 and FIG. 9 to FIG. 11, in some embodiments, the point-of-care testing device includes a control module 1 and at least one first point-of-care testing module 3.

The at least one first point-of-care testing module 3 may be one or two or more first point-of-care testing modules, and when the at least one first point-of-care testing module 3 is two or more first point-of-care testing modules, the first point-of-care testing modules may be of the same type or different types. Each first point-of-care testing module 3 has a first housing 30 and a first detection assembly 31, the first detection assembly 31 is mounted on the first housing 30, and the first detection assembly 31 is configured to perform detection on a sample. The control module 1 has a second housing 10 independent of the first housing 30 and a main control unit 11, and the main control unit 11 is mounted on the second housing 10. In addition to an electrical connection structure (for communication and/or power supply), the first housing 30 and the second housing 10, which are independent from each other, make the control module 1 and the first point-of-care testing module 3 relatively independent in physical space and may be separated from each other.

The main control unit 11 is communicatively connected to each of the first point-of-care testing modules 3, and the main control unit 11 is configured to receive a test request instruction input by a user and issue a control instruction to the corresponding first point-of-care testing module 3 according to the test request instruction; and each of the first point-of-care testing modules 3 is capable of completing at least one point-of-care testing operation under the control of the main control unit 11.

Referring to FIG. 1 to FIG. 5 and FIG. 12 to FIG. 14, some embodiments of the disclosure further provide another point-of-care testing device, which includes at least one first point-of-care testing module 3 and a second point-of-care testing module 2.

The second point-of-care testing module 2 has a second housing 10 independent of the first housing 30, a second detection assembly 21, and a main control unit 11. The second detection assembly 21 and the main control unit 11 are installed in the second housing 10. The second detection assembly 21 may be one or two or more second detection assemblies, and the second detection assemblies may be of the same type or different types. The main control unit 11 is communicatively connected to the second detection assembly 21, and controls, according to a test request instruction input by a user, the second detection assembly 21 to perform detection on a sample.

The main control unit 11 is further communicatively connected to each of the first point-of-care testing modules 3, and the main control unit 11 is configured to receive the test request instruction input by the user and issue a control instruction to the corresponding first point-of-care testing module 3 according to the test request instruction; and each of the first point-of-care testing modules 3 is capable of completing at least one point-of-care testing operation under the control of the main control unit 11.

Different from the control module 1 described above, in this embodiment, the second point-of-care testing module 2 can issue a control instruction to at least one first point-of-care testing module 3 by means of the main control unit 11, and the second point-of-care testing module 2 also has at least one second detection assembly 21, and may implement at least one point-of-care testing item under the control of the main control unit 11.

In the above-mentioned embodiments, the control module 1 or the second point-of-care testing module 2 may be used in combination with at least one first point-of-care testing module 3, allowing the user to select a first point-of-care testing module 3 having a required point-of-care testing item, such that the point-of-care testing module and the control module 1 or the second point-of-care testing module 2 form a point-of-care testing device. The control module 1 or the second point-of-care testing module 2 is configured to receive a test request instruction input by the user and issue a control instruction to the corresponding first point-of-care testing module 3 according to the test request instruction. Each first point-of-care testing module 3 is capable of completing at least one point-of-care testing operation under the control of the control module 1 or the second point-of-care testing module 2. The control module 1 or the second point-of-care testing module 2 may be used as a main control system to control one or more first point-of-care testing modules 3. The first point-of-care testing modules 3 may be simplified in structure provided that the first point-of-care testing modules may be used only to receive control instructions of the control module 1 or the second point-of-care testing module 2 and implement a corresponding point-of-care testing operation, which helps reduce the size of the entire point-of-care testing device. In addition, only a corresponding first point-of-care testing module 3 may be selected based on a required point-of-care testing function and then combined with the control module 1 or the second point-of-care testing module 2, helping to increase the diversity and availability of the point-of-care testing device and reduce the waste of the first point-of-care testing module 3.

In addition, the main control unit 11 of the control module 1 or the second point-of-care testing module 2 is capable of communicating with each first point-of-care testing module 3, so that different types of first point-of-care testing modules 3 in the point-of-care testing device may be replaced and types and a number of the first point-of-care testing modules 3 may be freely added or decreased according to actual requirements. For example, first point-of-care testing modules 3 that are not in use can be detached or not be assembled to reduce long-term space occupation of these first point-of-care testing modules 3 that are not in use for a long period of time. For another example, the user may correspondingly add a first point-of-care testing module 3 to extend a testing function of the point-of-care testing device, according to existing usage needs.

In the above-mentioned embodiments, the first detection assembly 31 and the second detection assembly 21 are used to test corresponding parameters of a sample, and the parameters of the sample tested by the first detection assembly 31 and the second detection assembly 21 may be different depending on different purposes of point-of-care testing. The first detection assembly 31 and the second detection assembly 21 may be various types of sensors or other components and structures capable of testing parameters of the sample. Depending on different point-of-care testing items, the first detection assembly 31 of the first point-of-care testing module 3 and the second detection assembly 21 of the second point-of-care testing module 2 that are of different types may be different or the same.

For example, in some embodiments, a type of the first point-of-care testing module 3 is at least one of an immunochromatographic detector, a microfluidic biochemical detector, a urine dry chemistry detector, a blood-gas and electrolyte detector, or a blood cell detector. Similarly, a type of the second point-of-care testing module 2 may also be at least one of an immunochromatographic detector, a microfluidic biochemical detector, a urine dry chemistry detector, a blood-gas and electrolyte detector, or a blood cell detector. In this case, the first detection assembly 31 in the corresponding first point-of-care testing module 3 and the second detection assembly 21 in the second point-of-care testing module 2 are also detection assemblies in corresponding detectors, and these detection assemblies may be implemented using an existing structure, which will not be described in detail herein.

In the above-mentioned embodiments, the first point-of-care testing module 3 and the second point-of-care testing module 2 may further have a sample pre-testing operation assembly 14. The sample pre-testing operation assembly 14 is used as an assembly to perform related operations on a sample, before the sample is added until the sample can be tested by the detection assemblies, and the sample pre-testing operation assembly may implement functions such as sample preparation, reagent/consumable preparation, incubation reaction, and/or state monitoring. For example, in some embodiments, the sample pre-testing operation assembly 14 may include an assembly that processes the sample as required into a processed sample capable of being tested by the first detection assembly 31 or the second detection assembly 21, may also include a scanning assembly to assist in identifying an identity of the sample or the reagent, and may further include a carrying assembly, etc. for carrying the sample or the reagent. In addition, the sample pre-testing operation assembly 14 may be an assembly that performs only some of operations of sample preparation, reagent/consumable preparation, incubation reaction, and/or state monitoring.

For example, in some embodiments, the first point-of-care testing module 3 and/or the second point-of-care testing module 2 are microfluidic biochemical detectors, and the sample needs to be subjected to centrifugal movement before detection. This allows the sample to mix with the corresponding diluent and flow to a set position of the microfluidic chip. In some embodiments, the sample pre-testing operation assembly 14 may include a disk centrifugal mechanism for centrifugally moving the microfluidic chip. Certainly, in some other embodiments, the sample pre-testing operation assembly 14 may further include a disc insertion/ejection mechanism (a mechanism for inserting and removing the microfluidic chip), a temperature control module (a module for constant-temperature control of the microfluidic chip), a calibration two-dimensional code reading module, etc.

For another example, in some embodiments, the first point-of-care testing module 3 and/or the second point-of-care testing module 2 is a fluorescence immunoassay point-of-care testing module, and the sample pre-testing operation assembly 14 may include a temperature control module (a module for constant-temperature control of a reagent card). In addition, in some embodiments, the sample pre-testing operation assembly 14 may further include a reagent card access mechanism (a mechanism for inserting and removing the reagent card), a scanning mechanism (driving an optical assembly to scan a test area of the reagent card), an ID card reading module (calibration information stored on an ID card), etc.

In summary, in different types of point-of-care testing modules (such as the first point-of-care testing module 3 and/or the second point-of-care testing module 2, the same below), the sample pre-testing operation assembly 14 may be configured to be different depending on different types. For a specific structure of the sample pre-testing operation assembly 14, refer to structures shown in various existing types of point-of-care testing devices.

Certainly, in some other embodiments, the sample pre-testing operation assembly 14 may also be omitted from the first point-of-care testing module 3 and the second point-of-care testing module 2, and these operations are performed manually or by using components other than the first point-of-care testing module 3 and the second point-of-care testing module 2.

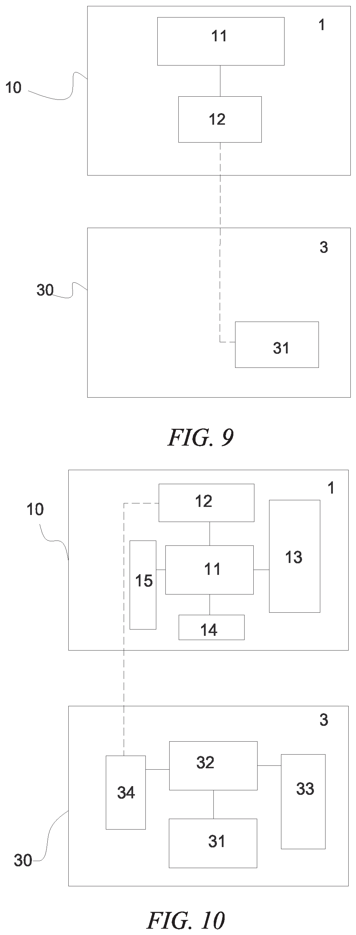

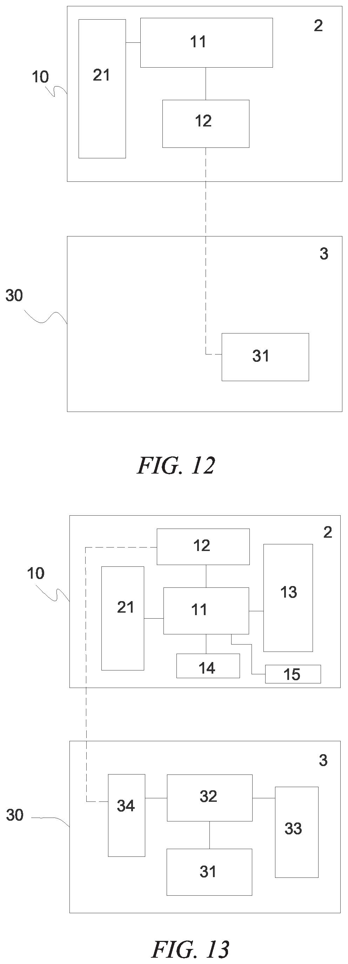

Further, there may be various manners of feeding back information to the control module 1 or the second point-of-care testing module 2 by the first point-of-care testing module 3. In some embodiments, referring to FIG. 9 and FIG. 12, the first detection assembly 31 includes a sensor configured to perform detection on the sample and obtain a detection signal, the first detection assembly 31 is capable of transmitting the detection signal to the main control unit 11, and the main control unit 11 processes the detection signal and obtains a detection result. That is, in this case, the first point-of-care testing module 3 does not have a control unit other than a control board configured by the first detection assembly 31. The obtained detection signal is directly sent by the first detection assembly 31 to the main control unit 11, and the main control unit 11 obtains the detection result based on the detection signal. For example, in some embodiments, a sensor used as the first detection assembly 31 is used to detect a specific physical or chemical or biological characteristic of a sample under test to generate a detection signal. The detection signal is sent directly to the main control unit 11, or is conditioned and acquired by a self-contained control board of the sensor and is converted into a digital signal to be sent to the main control unit 11. The sensor drives and supports a module being implemented by the self-contained control board of the sensor. In these embodiments, the first detection assembly 31 does not need to be specially provided with a control unit, and may further simplify the structure to reduce the size.

Alternatively, in some other embodiments, referring to FIG. 10 and FIG. 13, the first detection assembly 31 includes a sensor configured to perform detection on the sample and obtain a detection signal. The first point-of-care testing module 3 includes a sub-control unit 32 capable of processing the detection signal. The sub-control unit 32 obtains a detection result based on the detection signal, and the sub-control unit 32 is communicatively connected to the main control unit 11 and transmits the detection result to the main control unit 11. Specifically, a sensor used as the first detection assembly 31 is used to detect a specific physical or chemical or biological characteristic of a sample under test to generate a detection signal. The detection signal is sent directly to the sub-control unit 32. Alternatively, the detection signal is conditioned and acquired by a self-contained control board of the sensor and is converted into a digital signal to be sent to the sub-control unit 32. Then, the sub-control unit 32 calculates a result of a parameter corresponding to a characteristic quantity to be tested of the sample, and sends the result to the main control unit 11. In these embodiments, the first point-of-care testing module 3 may directly complete determination of the detection result, reducing a computing burden on the main control unit 11. Especially when a plurality of first point-of-care testing modules 3 are available at the same time, working efficiency of the main control unit 11 may be improved.

Alternatively, in some other embodiments, the above two manners of feeding back information to the control module 1 or the second point-of-care testing module 2 by the first point-of-care testing module 3 may be both included in the first point-of-care testing module 3.

Further, the control module 1 or the second point-of-care testing module 2 communicates with the first point-of-care testing module 3, as shown in FIG. 9, FIG. 10, FIG. 12, and FIG. 13, either in a wireless manner or in a wired manner, or in both of the manners. In this case, dashed lines in FIG. 9, FIG. 10, FIG. 12, and FIG. 13 only indicate that the control module 1 or the second point-of-care testing module 2 may communicate with the first point-of-care testing module 3, without limiting the specific form of communication.

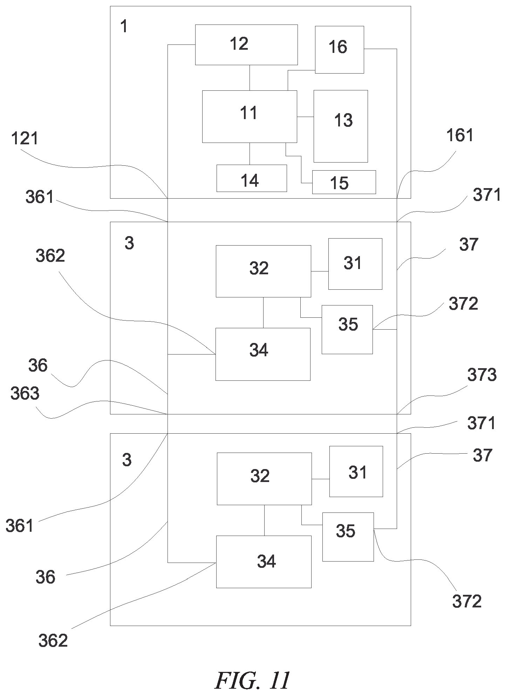

Referring to FIG. 11, in some embodiments, the control module 1 communicates with the first point-of-care testing module 3 in a wired manner. The first point-of-care testing module 3 has a communication unit 34 and a communication connection line 36, where the communication connection line 36 has a first communication port 361 and a second communication port 362, the first communication port 361 is configured to be supplied with a communication signal directly or indirectly output by the control module 1 and output a communication signal to the control module 1, and the second communication port 362 is electrically connected to the communication unit 34, such that the communication unit 34 is capable of communicating with the control module 1.

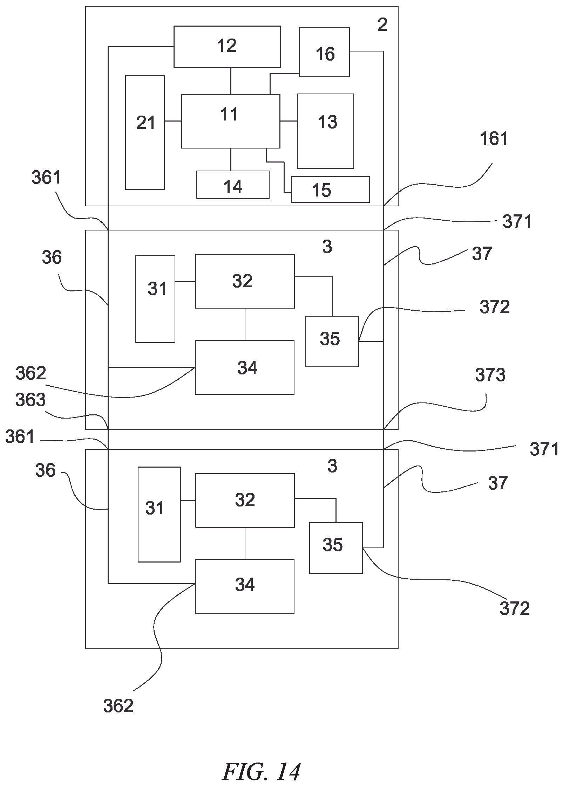

Similarly, in some embodiments, the second point-of-care testing module 2 may also communicate with the first point-of-care testing module 3 in a wired manner. Referring to FIG. 14, in this embodiment, at least part of the first point-of-care testing modules 3 has a communication unit 34 and a communication connection line 36, where the communication connection line 36 has a first communication port 361 and a second communication port 362, the first communication port 361 is configured to be supplied with a communication signal directly or indirectly output by the second point-of-care testing module 2 and output a communication signal to the second point-of-care testing module 2, and the second communication port 362 is electrically connected to the communication unit 34, such that the communication unit 34 is capable of communicating with the control module 1.

The communication signal directly or indirectly output by the control module 1 or the second point-of-care testing module 2 may come directly from the control module 1 or the second point-of-care testing module 2, or from another first point-of-care testing module 3 communicatively connected to the control module 1 or the second point-of-care testing module 2, that is, the another first point-of-care testing module 3 used as an intermediate transfer object is used to transfer the communication signal sent from the control module 1 or the second point-of-care testing module 2 to the first communication port 361 of the current first point-of-care testing module 3.

In some embodiments, in order to allow a first point-of-care testing module 3 to continue to extend backward to cover more first point-of-care testing modules 3, the first point-of-care testing module 3 may have a communication transfer function, such that the backward-extended first point-of-care testing modules 3 may be communicatively connected to the control module 1 or the second point-of-care testing module 2 through the current first point-of-care testing module 3.

With continued reference to FIG. 11 and FIG. 14, in some embodiments, at least part of the communication connection line 36 of the first point-of-care testing module 3 also has a third communication port 363, the third communication port 363 is electrically connected to the first communication port 361, and the third communication port 363 is configured to output the communication signal from the first communication port 361 to another first point-of-care testing module 3 and output a communication signal from the another first point-of-care testing module 3 to the first communication port 361. That is, the another first point-of-care testing module 3 may be communicatively connected to the control module 1 or the second point-of-care testing module 2 through the third communication port 363 to implement a serial connection, for example, using a daisy chain disclosed in the prior art. In this structure, by using the first point-of-care testing module 3 having the third communication port 363, a plurality of first point-of-care testing modules 3 may be connected in series in sequence to implement a series structure shown in FIG. 1 and FIG. 2. More first point-of-care testing modules 3 may be conveniently connected in series to form an integrated structure as needed, so that more flexibility is achieved in reassembling a point-of-care testing device, or a first point-of-care testing module 3 not commonly used may be detached, leaving only a commonly used first point-of-care testing module 3, making the entire point-of-care testing device smaller.

In addition, in other embodiments, each of the first point-of-care testing modules 3 may be communicatively connected to the control module 1 or the second point-of-care testing module 2 in other existing manners to exchange information. For example, in some embodiments, the first communication port 361 of each of the first point-of-care testing modules 3 is communicatively connected in parallel to the control module 1 or the second point-of-care testing module 2. Specifically, in some embodiments, each of the first point-of-care testing modules 3 may be communicatively connected to the control module 1 or the second point-of-care testing module 2, for example, in a commonly used star connection manner, through a respective connection cable.

Moreover, in addition to a wired manner, in some embodiments, the first point-of-care testing module 3 has a communication unit 34, and each communication unit 34 is connectable to the main control unit 11 via wireless communication. For example, the wireless communication is implemented using a Bluetooth technology, a SparkLink technology, a Wi-Fi technology, etc.

In some embodiments, the control module 1 or the second point-of-care testing module 2 is capable of independently communicating with each of the first point-of-care testing modules 3 without being affected by another first point-of-care testing module 3. That is, the control module 1 or the second point-of-care testing module 2 may separately control one or more first point-of-care testing modules 3, and the one or more first point-of-care testing modules 3 may also separately feed back a detection signal or a detection result to the control module 1 or the second point-of-care testing module 2. Thus, each first point-of-care testing module 3 may be separately detached from the integrated structure or assembled into the integrated structure, thereby implementing any combination and assembly between the control module 1 or the second point-of-care testing module 2 and the first point-of-care testing module 3.

Certainly, in some other embodiments, the control module 1 or the second point-of-care testing module 2 may alternatively synchronously control two or more first point-of-care testing modules 3. The disclosure does not set a limitation that the control module 1 or the second point-of-care testing module 2 can only communicate with each first point-of-care testing module 3 separately.

Further, in some embodiments, each first point-of-care testing module 3 may supply power through a self-contained power cable or a self-contained rechargeable battery. In some other embodiments, at least part of the first point-of-care testing modules 3 may alternatively be uniformly powered by the control module 1 or the second point-of-care testing module 2, which may further simplify the structure of the first point-of-care testing module 3 without the need to dispose an additional external power cable and adapter and a rechargeable battery.

Referring to FIG. 11, in some embodiments, at least part of the first point-of-care testing modules 3 has a power supply unit 35 and a power supply connection line 37, where the power supply connection line 37 has a first power supply port 371 and a second power supply port 372, the first power supply port 371 is configured to be connected to a power signal directly or indirectly output by the second point-of-care testing module 2, and the second power supply port 372 is in power supply connection to the power supply unit, to transmit the power signal directly or indirectly output by the second point-of-care testing module 2 to the power supply unit. The power signal is an electrical signal used to supply power to the first point-of-care testing module 3.

Similarly, referring to FIG. 14, in some embodiments, at least part of the first point-of-care testing modules 3 has a power supply unit 35 and a power supply connection line 37, where the power supply connection line 37 has a first power supply port 371 and a second power supply port 372, the first power supply port 371 is configured to be connected to a power signal directly or indirectly output by the second point-of-care testing module 2, and the second power supply port 372 is in power supply connection to the power supply unit, to transmit the power signal directly or indirectly output by the second point-of-care testing module 2 to the power supply unit.

The power signal directly or indirectly output by the control module 1 or the second point-of-care testing module 2 may come directly from the control module 1 or the second point-of-care testing module 2, or from another first point-of-care testing module 3 communicatively connected to the control module 1 or the second point-of-care testing module 2, that is, the another first point-of-care testing module 3 used as an intermediate transfer object is used to transfer the power signal sent from the control module 1 or the second point-of-care testing module 2 to the first power supply port 371 of the current first point-of-care testing module 3.

Further, in some embodiments, in order to allow a first point-of-care testing module 3 to continue to supply power to more other first point-of-care testing modules 3, at least part of the first point-of-care testing modules 3 may have a power-supply transfer function, such that the backward-extended first point-of-care testing modules 3 may be in power supply connection to the control module 1 or the second point-of-care testing module 2 through the current first point-of-care testing module 3.

With continued reference to FIG. 11 and FIG. 14, in some embodiments, at least part of the power supply connection line 37 of the first point-of-care testing module 3 also has a third power supply port 373, the third power supply port 373 is in power supply connection to the first power supply port 371, and the third power supply port 373 is configured to output a power signal of the first power supply port 371 to a next first point-of-care testing module 3. That is, the another first point-of-care testing module 3 may be in power supply connection to the control module 1 or the second point-of-care testing module 2 through the third power supply port 373 to implement a serial connection, for example, using a daisy chain disclosed in the prior art. In this structure, by using the first point-of-care testing module 3 having the third power supply port 373, a plurality of first point-of-care testing modules 3 may be connected in series in sequence to implement a series power supply structure shown in FIG. 1 and FIG. 2.

In addition, in other embodiments, each of the first point-of-care testing modules 3 may be in power supply connection to the control module 1 or the second point-of-care testing module 2 in other manners, and is uniformly powered by the control module 1 or the second point-of-care testing module 2. For example, in some embodiments, each of the first point-of-care testing modules 3 may be in power supply connection to the control module 1 or the second point-of-care testing module 2, for example, in a commonly used star connection manner, through a respective power connection cable.

Moreover, in addition to a wired manner, in some embodiments, the control module 1 or the second point-of-care testing module 2 may charge some or all of first point-of-care testing modules 3 in a wireless charging manner. As a further extension, some or all of the first point-of-care testing modules 3 may also be used as power sources for providing wireless charging or wired power supply to other first point-of-care testing modules 3.

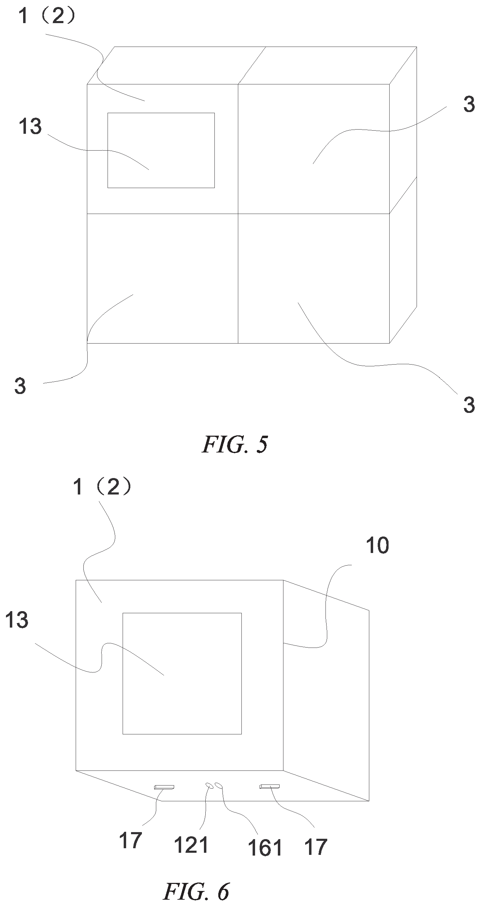

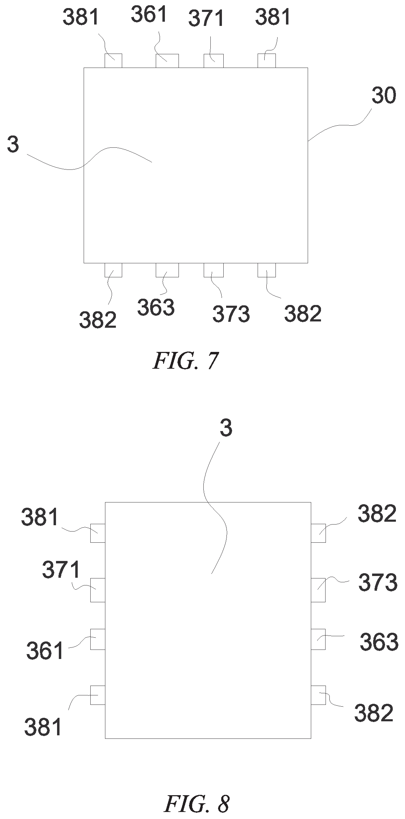

Further, to form an integrated structure that is stacked and assembled vertically, referring to FIG. 6 and FIG. 7, in some embodiments, a main communication port 121 and/or a main power supply port 161, which are used for docking with the first point-of-care testing module 3, on the control module 1 or the second point-of-care testing module 2 may be disposed on a bottom wall of the second housing 10. For the first point-of-care testing module 3, the first communication port 361 and/or the first power supply port 371 is disposed on a top wall of the first housing 30. During assembling, the control module 1 or the second point-of-care testing module 2 may be placed above the first point-of-care testing module 3, so that the main communication port 121 is docked with the first communication port 361 and the main power supply port 161 is docked with the first power supply port 371. This structure not only allows the control module 1 or the second point-of-care testing module 2 and the corresponding first point-of-care testing module 3 to form a vertical structure, but also ensures that the control module 1 or the second point-of-care testing module 2 is located above the first point-of-care testing module 3, such that the user may control the control module 1 or the second point-of-care testing module 2.

In order to enable first point-of-care testing modules 3 at a back end to be stacked and assembled vertically, in some embodiments, referring to FIG. 7, for the first point-of-care testing module 3, the first communication port 361 and the third communication port 363 are disposed opposite each other, with the first communication port 361 located on the top wall of the first housing 30 and the third communication port 363 located on a bottom wall of the first housing 30; and/or the first power supply port 371 and the third power supply port 373 are disposed opposite each other, with the first power supply port 371 located on the top wall of the first housing 30 and the third power supply port 373 located on the bottom wall of the first housing 30. In this way, the control module 1 or the second point-of-care testing module 2 and one or two or more first point-of-care testing modules 3 may form a vertical arrangement combination as shown in FIG. 1. The vertical arrangement combination may minimize the occupation of the lateral space and make full use of the space, allowing for extension to cover more first point-of-care testing modules 3 and implement a set of more timely testing functions, without increasing a lateral area of the entire point-of-care testing device.

Certainly, the settings of the ports are not limited to those shown in FIG. 6 and FIG. 7, and in other embodiments, the main communication port 121 and/or the main power supply port 161 on the control module 1 or the second point-of-care testing module 2 may also be disposed on a top wall or a circumferential side wall of the second housing 10. All outer walls of the first housing 30, such as a top wall, a bottom wall, and a side wall, may be used to dispose communication ports and/or power supply ports. As shown in FIG. 8, in some embodiments, the communication ports and/or power supply ports may alternatively be arranged on a left side wall and a right side wall of the first housing 30.

Further, in some embodiments, referring to FIG. 7 and FIG. 8, the first point-of-care testing module 3 has a first pogo pin interface, and the first pogo pin interface includes the first communication port 361 and the first power supply port 371.

In some embodiments, with continued reference to FIG. 7 and FIG. 8, the first point-of-care testing module 3 has a second pogo pin interface, and the second pogo pin interface includes the third communication port 363 and the third power supply port 373.

Accordingly, a main communication port 121 and/or a main power supply port 161, which are used for docking with the first point-of-care testing module 3, on the control module 1 or the second point-of-care testing module 2, may also be, but is not limited to, a pogo pin interface.

In the above-mentioned embodiments, the pogo pin interface implements a convenient, fast, and stable-docking electrical connection structure. Certainly, in other embodiments, the first communication port 361 and the first power supply port 371, and the third communication port 363 and the third power supply port 373 may alternatively use other forms of wired electrical connection docking structures, which are not limited to the pogo pin interface.

In addition to the above-mentioned communication connection and power connection, the control module 1 or the second point-of-care testing module 2 and the corresponding first point-of-care testing module 3 may alternatively be assembled in space to form an integrated structure.

For example, referring to FIG. 1 to FIG. 6, in some embodiments, the control module 1 or the second point-of-care testing module 2 has an external mounting structure 17, and the external mounting structure 17 is detachably connected to the corresponding first point-of-care testing module 3, such that the control module 1 is assemblable with the corresponding first point-of-care testing module 3 to form an integrated structure. Accordingly, referring to FIG. 7, in some embodiments, the first point-of-care testing module 3 may have a first mounting structure 381. The first mounting structure 381 may be detachably connected to the external mounting structure 17 of the control module 1 or the second point-of-care testing module 2 to mount the corresponding first point-of-care testing module 3 to the control module 1 or the second point-of-care testing module 2.

When there are at least two first point-of-care testing modules 3, in order to extend to cover more first point-of-care testing modules 3 from some first point-of-care testing modules 3, referring to FIG. 7, in some embodiments, at least one of the first point-of-care testing modules 3 has a first mounting structure 381 and a second mounting structure 382, and the first mounting structure 381 is detachably connectable to the external mounting structure 17 of the control module 1 or a second mounting structure 382 of another first point-of-care testing module 3. That is, as a first point-of-care testing module 3 that is extended from the current first point-of-care testing module 3, the first mounting structure 381 of the extended first point-of-care testing module 3 may be detachably connected to a second mounting structure 382 of a previous first point-of-care testing module 3.

In some embodiments, the detachable connection between the first mounting structure 381 and the external mounting structure 17 and/or the detachable connection between the first mounting structure 381 and the second mounting structure 382 may be at least one of snap fit, screw connection, magnetic fixation, fastener fixation, or adhesive bonding, provided that a connection may be made between the control module 1 or the second point-of-care testing module 2 and the first point-of-care testing module 3 and between different first point-of-care testing modules 3 to form an integrated structure.

In some embodiments, referring to FIG. 6 and FIG. 7, to form an integrated structure that is stacked and assembled vertically, a bottom wall of the second housing 10 is provided with the external mounting structure 17, and the first mounting structure 381 is disposed on the top wall of the first housing 30 of the first point-of-care testing module 3, such that the control module 1 or the second point-of-care testing module 2 is capable of being located above the first point-of-care testing module 3.

In order to enable first point-of-care testing modules 3 at a back end to be stacked and assembled vertically, in some embodiments, referring to FIG. 7, the second mounting structure 382 is disposed on a bottom wall of the first point-of-care testing module 3, such that each first point-of-care testing module 3 and the control module 1 or the second point-of-care testing module 2 may be stacked vertically, as shown in FIG. 1.

Certainly, in other embodiments, the external mounting structure 17 may also be disposed on another outer wall of the second housing 10, for example, on the top wall or the circumferential side wall. In other embodiments, the external mounting structure 17 on the control module 1 or the second point-of-care testing module 2 may also be disposed on the top wall or the circumferential side wall of the second housing 10. All outer walls of the first housing 30, such as a top wall, a bottom wall, and a side wall, may be used to dispose the first mounting structure 381 and the second mounting structure 382.

In addition, in addition to forming a mechanical connection structure, in some other embodiments, the control module 1 or the second point-of-care testing module 2 and each of the first point-of-care testing modules 3 are stacked into an integrated structure in a non-mechanical connection manner. That is, except for a necessary electrical connection structure, there is no mechanical connection structure between the control module 1 or the second point-of-care testing module 2 and each of the first point-of-care testing modules 3, which are stacked to form various integrated structures. The shapes of the integrated structures may be, but are not limited to, being shown in FIG. 1 to FIG. 5.

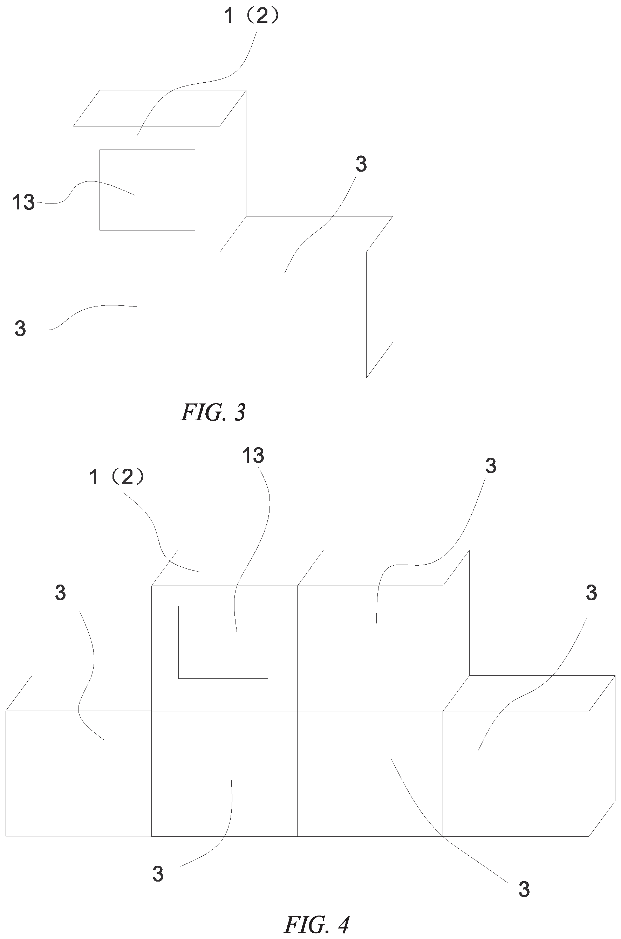

Whether there is a connection having a mechanical connection relationship (for example, a detachable connection) or a direct stacking having no mechanical connection relationship, a shape obtained after the control module 1 or the second point-of-care testing module 2 is stacked and assembled with each of the first point-of-care testing modules 3 is shown in some embodiments. For example, as shown in FIG. 1, the control module 1 or the second point-of-care testing module 2 and each of the first point-of-care testing modules 3 may be stacked vertically; or as shown in FIG. 2, the control module 1 or the second point-of-care testing module 2 and each of the first point-of-care testing modules 3 are arranged in a straight line; or as shown in FIG. 3, the control module 1 or the second point-of-care testing module 2 and each of the first point-of-care testing modules 3 are stacked in a triangular shape; or as shown in FIG. 4, the control module 1 or the second point-of-care testing module 2 and each of the first point-of-care testing modules 3 are stacked in a pyramidal shape; or as shown in FIG. 5, the control module 1 or the second point-of-care testing module 2 and each of the first point-of-care testing modules 3 are stacked in a cubic shape. In addition, the control module 1 or the second point-of-care testing module 2 and each of the first point-of-care testing modules 3 are not necessarily stacked and assembled in the illustrated shapes, but may also be in other shapes, e.g., in a staggered triangular shape.

In some embodiments, in the integrated structure, the control module 1 or the second point-of-care testing module 2 is located at the top of the integrated structure, regardless of the manner in which assembly and combination are performed. In this way, the control module 1 or the second point-of-care testing module 2 is used to form a specific height by stacking the first point-of-care testing module 3, thus helping the user to control the control module 1 or the second point-of-care testing module 2 and view information from the control module 1 or the second point-of-care testing module 2.

In particular, in some embodiments, referring to FIG. 10 and FIG. 14, the control module 1 or the second point-of-care testing module 2 may also have an auxiliary functional component. For example, the auxiliary functional component includes, but is not limited to, at least one of an external power input interface 16, a human-machine interaction unit 13, and a printer 15. The external power input interface 16 allows the control module 1 or the second point-of-care testing module 2 to uniformly power some or all of first point-of-care testing modules 3, simplifying the first point-of-care testing modules 3. The human-machine interaction unit 13 may include, but is not limited to, a display screen, a touch screen, a touch button, a physical button, or other information interaction structures. No human-machine interaction unit 13 may be disposed on the another first point-of-care testing module 3, and the user controls each first point-of-care testing module 3 through the human-machine interaction unit 13 of the control module 1 or the second point-of-care testing module 2 and views examination results of each first point-of-care testing module 3. The structure of the first point-of-care testing module 3 is further simplified. The printer 15 may be configured to print various types of information, such as the detection results of each first point-of-care testing module 3, when required. In this embodiment, by integrating various auxiliary functional components into the control module 1 or the second point-of-care testing module 2 and using these auxiliary functional components to use the principle of component sharing for the first point-of-care testing module 3, this further simplifies the structure of each first point-of-care testing module 3, making the entire point-of-care testing device smaller and occupying less space, while also helping the user to control and view results.

Although the principles herein are shown in various embodiments, many modifications of structures, arrangements, proportions, elements, materials, and components particularly applicable to specific environmental and operating requirements may be made without departing from the principles and scope of the disclosure. These modifications and other changes or corrections fall within the scope of this specification.

The foregoing detailed descriptions are provided with reference to various embodiments. However, those skilled in the art should understand that various corrections and changes may be made without departing from the scope of the disclosure. Therefore, the disclosure is intended for an illustrative purpose other than a limiting purpose, and all these modifications fall within the scope of the disclosure. Also, the advantages, other advantages and the solutions to problems related to various embodiments have been described above. However, the benefits, the advantages, the solutions to the problems, and any of their contributing factors, or solutions clarifying them should not be construed to be critical, necessary, or essential. The term "include" used herein and any other variations thereof all refer to a non-exclusive inclusion, such that a process, method, article, or device including a list of elements includes not only these elements, but also other elements that are not expressly listed or not inherent to the process, method, system, article, or device. In addition, the term "couple" used herein and any other variations thereof refer to a physical connection, an electrical connection, a magnetic connection, an optical connection, a communication connection, a functional connection, and/or any other connections.

Those skilled in the art should understand that many changes may be made to the details of the foregoing embodiments without departing from the basic principles of the disclosure. Therefore, the scope of the disclosure should be determined in accordance with the following claims.

## Claims

1. A point-of-care testing device, **characterized by** comprising:
at least one first point-of-care testing module, each of which has a first housing and a first detection assembly, the first detection assembly being mounted on the first housing, and the first detection assembly being configured to perform detection on a sample;
and a control module having a second housing independent of the first housing and a main control unit, the main control unit being mounted on the second housing, wherein
the main control unit is communicatively connected to each of the first point-of-care testing modules, and is configured to receive a test request instruction input by a user and issue a control instruction to the corresponding first point-of-care testing module according to the test request instruction; and each of the first point-of-care testing modules is capable of completing at least one point-of-care testing operation under the control of the main control unit.

2. The point-of-care testing device of claim 1, **characterized in that** the first detection assembly comprises a sensor configured to perform detection on the sample and obtain a detection signal, the first detection assembly is capable of transmitting the detection signal to the main control unit, and the main control unit processes the detection signal and obtains a detection result; or
the first detection assembly comprises a sensor configured to perform detection on the sample and obtain a detection signal and a sub-control unit capable of processing the detection signal, the sub-control unit obtains a detection result based on the detection signal, the sub-control unit is communicatively connected to the main control unit, and the sub-control unit transmits the detection result to the main control unit.

3. The point-of-care testing device of claim 1 or 2, **characterized in that** the first point-of-care testing module has a communication unit and a communication connection line, wherein the communication connection line has a first communication port and a second communication port, the first communication port is configured to be supplied with a communication signal directly or indirectly output by the control module and output a communication signal to the control module, and the second communication port is electrically connected to the communication unit, such that the communication unit is capable of communicating with the control module.

4. The point-of-care testing device of claim 3, **characterized in that** at least part of the communication connection line also has a third communication port, the third communication port is electrically connected to the first communication port, and the third communication port is configured to output the communication signal from the first communication port to another first point-of-care testing module and output a communication signal from the another first point-of-care testing module to the first communication port.

5. The point-of-care testing device of claim 3 or 4, **characterized in that** at least part of the first point-of-care testing modules has a power supply unit and a power supply connection line, wherein the power supply connection line has a first power supply port and a second power supply port, the first power supply port is configured to be supplied with a power signal directly or indirectly output by the control module, and the second power supply port is in power supply connection to the power supply unit, to transmit the power signal directly or indirectly output by the control module to the power supply unit.

6. The point-of-care testing device of claim 5, **characterized in that** at least part of the power supply connection line also has a third power supply port, the third power supply port is in power supply connection to the first power supply port, and the third power supply port is configured to output a power signal of the first power supply port to a next first point-of-care testing module.

7. The point-of-care testing device of claim 3, **characterized in that** the first communication port of each of the first point-of-care testing modules is communicatively connected in parallel to the control module.

8. The point-of-care testing device of claim 7, **characterized in that** each of the first point-of-care testing modules has a power supply unit and a power supply connection line, wherein the power supply connection line has a first power supply port and a second power supply port, the second power supply port is in power supply connection to the power supply unit, and the first power supply port of each of the first point-of-care testing modules is electrically connected in parallel to the control module.

9. The point-of-care testing device of any one of claims 1 to 8, **characterized in that** for the first point-of-care testing module, the first communication port and/or the first power supply port is disposed on a top wall of the first housing.

10. The point-of-care testing device of any one of claims 1 to 8, **characterized in that** for the first point-of-care testing module, the first communication port and the third communication port are disposed opposite each other, with the first communication port located on the top wall of the first housing and the third communication port located on a bottom wall of the first housing; and/or
the first power supply port and the third power supply port are disposed opposite each other, with the first power supply port located on the top wall of the first housing and the third power supply port located on the bottom wall of the first housing.

11. The point-of-care testing device of any one of claims 1 to 10, **characterized in that** the first point-of-care testing module has a first pogo pin interface, and the first pogo pin interface comprises the first communication port and the first power supply port.

12. The point-of-care testing device of claim 11, **characterized in that** the first point-of-care testing module has a second pogo pin interface, and the second pogo pin interface comprises the third communication port and the third power supply port.

13. The point-of-care testing device of claim 1 or 2, **characterized in that** the first point-of-care testing module has a communication unit, and each communication unit is connectable to the main control unit via wireless communication.

14. The point-of-care testing device of any one of claims 1 to 13, **characterized in that** the control module is capable of independently communicating with each of the first point-of-care testing modules.

15. The point-of-care testing device of any one of claims 1 to 14, **characterized in that** the control module has an external mounting structure, and the external mounting structure is detachably connected to the corresponding first point-of-care testing module, such that the control module is assemblable with the corresponding first point-of-care testing module to form an integrated structure.

16. The point-of-care testing device of claim 14 or 15, **characterized in that** there are at least two first point-of-care testing modules, at least one of the first point-of-care testing modules has a first mounting structure and a second mounting structure, and the first mounting structure is detachably connectable to the external mounting structure of the control module or the second mounting structure of another first point-of-care testing module.

17. The point-of-care testing device of claim 16, **characterized in that** a bottom wall of the second housing is provided with the external mounting structure, and the first mounting structure is disposed on the top wall of the first housing of the first point-of-care testing module, such that the control module is capable of being located above the first point-of-care testing module.

18. The point-of-care testing device of claim 17, **characterized in that** the second mounting structure is disposed on the bottom wall of the first point-of-care testing module for docking with the first mounting structure of another first point-of-care testing module, such that each of the first point-of-care testing modules is stackable vertically with the control module.

19. The point-of-care testing device of any one of claims 15 to 18, **characterized in that** the detachable connection is at least one of snap fit, screw connection, magnetic fixation, fastener fixation, or adhesive bonding.

20. The point-of-care testing device of any one of claims 1 to 13, **characterized in that** the control module and each of the first point-of-care testing modules are stacked into an integrated structure in a non-mechanical connection manner.

21. The point-of-care testing device of claim 14 or 20, **characterized in that** in the integrated structure, the control module is located at the top of the integrated structure.

22. The point-of-care testing device of claim 14 or 20, **characterized in that** the control module and each of the first point-of-care testing modules are stacked vertically;
or the control module and each of the first point-of-care testing modules are stacked in a pyramidal shape;
or the control module and each of the first point-of-care testing modules are stacked in a cubic shape;
or the control module and each of the first point-of-care testing modules are arranged in a straight line.

23. The point-of-care testing device of any one of claims 1 to 22, **characterized in that** the control module has at least one of an external power input interface, a human-machine interaction unit, or a printer.

24. The point-of-care testing device of any one of claims 1 to 23, **characterized in that** at least one of the first point-of-care testing modules has a built-in power supply battery.

25. The point-of-care testing device of any one of claims 1 to 24, **characterized in that** a type of the first point-of-care testing module is at least one of an immunochromatographic detector, a microfluidic biochemical detector, a urine dry chemistry detector, a blood-gas and electrolyte detector, or a blood cell detector.

26. A point-of-care testing device, **characterized by** comprising:
at least one first point-of-care testing module, each of which has a first housing and a first detection assembly, the first detection assembly being mounted on the first housing, and the first detection assembly being configured to perform detection on a sample;
and a second point-of-care testing module having a second housing independent of the first housing, a second detection assembly, and a main control unit, the second detection assembly and the main control unit being mounted on the second housing, and the main control unit being communicatively connected to the second detection assembly and controlling, according to a test request instruction input by a user, the second detection assembly to perform detection on the sample, wherein
the main control unit is further communicatively connected to each of the first point-of-care testing modules, and is configured to receive the test request instruction input by the user and issue a control instruction to the corresponding first point-of-care testing module according to the test request instruction; and each of the first point-of-care testing modules is capable of completing at least one point-of-care testing operation under the control of the main control unit.

27. The point-of-care testing device of claim 26, **characterized in that** the first detection assembly comprises a sensor configured to perform detection on the sample and obtain a detection signal, the first detection assembly is capable of transmitting the detection signal to the main control unit, and the main control unit processes the detection signal and obtains a detection result; or
the first detection assembly comprises a sensor configured to perform detection on the sample and obtain a detection signal and a sub-control unit capable of processing the detection signal, the sub-control unit obtains a detection result based on the detection signal, the sub-control unit is communicatively connected to the main control unit, and the sub-control unit transmits the detection result to the main control unit.

28. The point-of-care testing device of claim 26 or 27, **characterized in that** the first point-of-care testing module has a communication unit and a communication connection line, wherein the communication connection line has a first communication port and a second communication port, the first communication port is configured to be supplied with a communication signal directly or indirectly output by the second point-of-care testing module and output a communication signal to the second point-of-care testing module, and the second communication port is electrically connected to the communication unit, such that the communication unit is capable of communicating with the control module.

29. The point-of-care testing device of claim 28, **characterized in that** at least part of the communication connection line also has a third communication port, the third communication port is electrically connected to the first communication port, and the third communication port is configured to output the communication signal from the first communication port to another first point-of-care testing module and output a communication signal from the another first point-of-care testing module to the first communication port.

30. The point-of-care testing device of claim 29, **characterized in that** at least part of the first point-of-care testing modules has a power supply unit and a power supply connection line, wherein the power supply connection line has a first power supply port and a second power supply port, the first power supply port is configured to be supplied with a power signal directly or indirectly output by the second point-of-care testing module, and the second power supply port is in power supply connection to the power supply unit, to transmit the power signal directly or indirectly output by the second point-of-care testing module to the power supply unit.

31. The point-of-care testing device of claim 30, **characterized in that** at least part of the power supply connection line also has a third power supply port, the third power supply port is in power supply connection to the first power supply port, and the third power supply port is configured to output a power signal of the first power supply port to a next first point-of-care testing module.

32. The point-of-care testing device of claim 28, **characterized in that** the first communication port of each of the first point-of-care testing modules is communicatively connected in parallel to the second point-of-care testing module.

33. The point-of-care testing device of claim 32, **characterized in that** each of the first point-of-care testing modules has a power supply unit and a power supply connection line, wherein the power supply connection line has a first power supply port and a second power supply port, the second power supply port is in power supply connection to the power supply unit, and the first power supply port of each of the first point-of-care testing modules is electrically connected in parallel to the second point-of-care testing module.

34. The point-of-care testing device of any one of claims 26 to 33, **characterized in that** for the first point-of-care testing module, the first communication port and/or the first power supply port is disposed on a top wall of the first housing.

35. The point-of-care testing device of any one of claims 26 to 33, **characterized in that** for the first point-of-care testing module, the first communication port and the third communication port are disposed opposite each other, with the first communication port located on the top wall of the first housing and the third communication port located on a bottom wall of the first housing; and/or
the first power supply port and the third power supply port are disposed opposite each other, with the first power supply port located on the top wall of the first housing and the third power supply port located on the bottom wall of the first housing.

36. The point-of-care testing device of any one of claims 26 to 35, **characterized in that** the first point-of-care testing module has a first pogo pin interface, and the first pogo pin interface comprises the first communication port and the first power supply port.

37. The point-of-care testing device of claim 36, **characterized in that** the first point-of-care testing module has a second pogo pin interface, and the second pogo pin interface comprises the third communication port and the third power supply port.

38. The point-of-care testing device of claim 26 or 27, **characterized in that** the first point-of-care testing module has a communication unit, and each communication unit is connectable to the main control unit via wireless communication.

39. The point-of-care testing device of any one of claims 26 to 38, **characterized in that** the second point-of-care testing module is capable of independently communicating with each of the first point-of-care testing modules.

40. The point-of-care testing device of any one of claims 26 to 39, **characterized in that** the second point-of-care testing module has an external mounting structure, and the external mounting structure is detachably connected to the corresponding first point-of-care testing module, such that the second point-of-care testing module is assemblable with the corresponding first point-of-care testing module to form an integrated structure.

41. The point-of-care testing device of claim 39 or 40, **characterized in that** there are at least two first point-of-care testing modules, at least one of the first point-of-care testing modules has a first mounting structure and a second mounting structure, and the first mounting structure is detachably connectable to the external mounting structure of the second point-of-care testing module or the second mounting structure of another first point-of-care testing module.

42. The point-of-care testing device of claim 41, **characterized in that** a bottom wall of the second housing is provided with the external mounting structure, and the first mounting structure is disposed on the top wall of the first housing of the first point-of-care testing module, such that the second point-of-care testing module is capable of being located above the first point-of-care testing module.

43. The point-of-care testing device of claim 42, **characterized in that** the second mounting structure is disposed on the bottom wall of the first point-of-care testing module for docking with the first mounting structure of another first point-of-care testing module, such that each of the first point-of-care testing modules is stackable vertically with the second point-of-care testing module.

44. The point-of-care testing device of any one of claims 30 to 43, **characterized in that** the detachable connection is at least one of snap fit, screw connection, magnetic fixation, fastener fixation, or adhesive bonding.

45. The point-of-care testing device of any one of claims 26 to 38, **characterized in that** the second point-of-care testing module and each of the first point-of-care testing modules are stacked into an integrated structure in a non-mechanical connection manner.

46. The point-of-care testing device of claim 29 or 45, **characterized in that** in the integrated structure, the second point-of-care testing module is located at the top of the integrated structure.

47. The point-of-care testing device of claim 29 or 45, **characterized in that** the second point-of-care testing module and each of the first point-of-care testing modules are stacked vertically;
or the second point-of-care testing module and each of the first point-of-care testing modules are stacked in a pyramidal shape;
or the second point-of-care testing module and each of the first point-of-care testing modules are stacked in a cubic shape;
or the second point-of-care testing module and each of the first point-of-care testing modules are arranged in a straight line.

48. The point-of-care testing device of any one of claims 26 to 47, **characterized in that** the second point-of-care testing module has at least one of an external power input interface, a human-machine interaction unit, or a printer.

49. The point-of-care testing device of any one of claims 26 to 48, **characterized in that** at least one of the first point-of-care testing modules has a built-in power supply battery.

50. The point-of-care testing device of any one of claims 26 to 49, **characterized in that** a type of the first point-of-care testing module is at least one of an immunochromatographic detector, a microfluidic biochemical detector, a urine dry chemistry detector, a blood-gas and electrolyte detector, or a blood cell detector.

51. A control module of a point-of-care testing device, **characterized by** having a main control unit, a main communication unit, and a second housing disposed independent of a first point-of-care testing module, wherein the main control unit and the main communication unit are mounted on the second housing; and the main communication unit is configured to be communicatively connected to a corresponding first point-of-care testing module, and the main control unit is configured to receive a test request instruction input by a user and is capable of issuing a control instruction to the corresponding first point-of-care testing module according to the test request instruction.

52. The control module of claim 51, **characterized in that** the second housing has an external mounting structure, and the external mounting structure is detachably connectable to the corresponding point-of-care testing module, such that the control module is assemblable with the corresponding point-of-care testing module to form an integrated structure.

53. A second point-of-care testing module of a point-of-care testing device, **characterized by** having a second housing, a second detection assembly, a main communication unit, and a main control unit, wherein the second detection assembly, the main communication unit, and the main control unit are mounted on the second housing, and the second detection assembly is configured to perform detection on a sample; and the main communication unit is configured to be communicatively connected to a corresponding first point-of-care testing module, and the main control unit is configured to receive a test request instruction input by a user and is capable of issuing a control instruction to the corresponding first point-of-care testing module according to the test request instruction.

54. The control module of claim 53, **characterized in that** the second housing has an external mounting structure, and the external mounting structure is detachably connectable to the corresponding point-of-care testing module, such that the control module is assemblable with the corresponding point-of-care testing module to form an integrated structure.

55. A first point-of-care testing module of a point-of-care testing device, **characterized by** having a first housing, a communication unit, and a first detection assembly, wherein the first detection assembly is configured to perform detection on a sample, and the communication unit is configured to be communicatively connected to a main control unit; and
the first detection assembly and the communication unit are mounted on the first housing; and the first housing has a first mounting structure, and the first mounting structure is detachably connectable to the control module or the second point-of-care testing module, such that the first point-of-care testing module is assembled with the control module or the second point-of-care testing module to form an integrated structure.

56. The first point-of-care testing module of claim 55, **characterized in that** the first housing has a second mounting structure capable of extension by connecting another first point-of-care testing module, and the second mounting structure is configured to assemble the first point-of-care testing module and the another first point-of-care testing module into an integrated structure.

57. The first point-of-care testing module of claim 56, **characterized in that** the first mounting structure and the second mounting structure are respectively disposed on two opposite side surfaces of the first housing.

58. The first point-of-care testing module of any one of claims 55 to 57, **characterized in that** the first point-of-care testing module has a communication connection line, wherein the communication connection line has a first communication port and a second communication port, the first communication port is configured to be supplied with a communication signal directly or indirectly output by the second point-of-care testing module and output a communication signal to the second point-of-care testing module, and the second communication port is electrically connected to the communication unit, such that the communication unit is capable of communicating with the main control unit.

59. The first point-of-care testing module of claim 58, **characterized in that** the communication connection line also has a third communication port, the third communication port is electrically connected to the first communication port, and the third communication port is configured to output the communication signal from the first communication port to another first point-of-care testing module and output a communication signal from the another first point-of-care testing module to the first communication port.

60. The first point-of-care testing module of claim 58 or 59, **characterized in that** the first point-of-care testing module has a power supply unit and a power supply connection line, wherein the power supply connection line has a first power supply port and a second power supply port, the first power supply port is configured to be supplied with a power signal directly or indirectly output by the control module, and the second power supply port is in power supply connection to the power supply unit, to transmit the power signal directly or indirectly output by the control module to the power supply unit.

61. The first point-of-care testing module of claim 60, **characterized in that** at least part of the power supply connection line also has a third power supply port, the third power supply port is in power supply connection to the first power supply port, and the third power supply port is configured to output a power signal of the first power supply port to a next first point-of-care testing module.

62. The first point-of-care testing module of any one of claims 55 to 61, **characterized in that** for the first point-of-care testing module, the first communication port and/or the first power supply port is disposed on a top wall of the first housing.

63. The first point-of-care testing module of any one of claims 55 to 61, **characterized in that** for the first point-of-care testing module, the first communication port and the third communication port are disposed opposite each other, with the first communication port located on the top wall of the first housing and the third communication port located on a bottom wall of the first housing; and/or
the first power supply port and the third power supply port are disposed opposite each other, with the first power supply port located on the top wall of the first housing and the third power supply port located on the bottom wall of the first housing.

64. The first point-of-care testing module of any one of claims 55 to 63, **characterized in that** the first point-of-care testing module has a first pogo pin interface, and the first pogo pin interface comprises the first communication port and the first power supply port.

65. The first point-of-care testing module of claim 64, **characterized in that** the first point-of-care testing module has a second pogo pin interface, and the second pogo pin interface comprises the third communication port and the third power supply port.
